# EUROPÄISCHE PATENTANMELDUNG

(11) **EP 4 113 227 A1**
(43) Veröffentlichungstag der Anmeldung: **04.01.2023**
(21) Anmeldenummer: 21183074.0
(22) Anmeldetag: 01.07.2021
(51) Int. Cl.: G05B 19/12

(54) **BEARBEITUNGSGERÄT ZUM BEARBEITEN EINER DENTALEN INDIKATION**

(71) Anmelder: Ivoclar Vivadent AG, 9494 Schaan (LI)
(72) Erfinder: JUSSEL, Rudolf, 6805 Gisingen (AT); SENTI, Theresa Sujata Maria, 9497 Triesenberg (LI)
(74) Vertreter: Baldus, Oliver

(57) **Zusammenfassung**

Die vorliegende Erfindung betrifft ein Bearbeitungsgerät (100) zum Bearbeiten einer dentalen Indikation (103), mit einer elektronischen Zustandserfassungsvorrichtung (105) zum Erfassen von Zustandsdaten des Bearbeitungsgeräts (100) und/oder der dentalen Indikation (103); und einer elektronischen Erzeugungsvorrichtung (107) zum Erzeugen eines Codes (109), der die Zustandsdaten umfasst.

## Beschreibung

Die vorliegende Erfindung betrifft ein Bearbeitungsgerät zum Bearbeiten einer dentalen Indikation, ein Bearbeitungssystem mit dem Bearbeitungsgerät und ein Verfahren zum Bearbeiten einer dentalen Indikation.

Aktuell werden Geräteinformationen bei der Bearbeitung von dentalen Indikationen manuell verwaltet. Ein Zahntechniker oder Zahnarzt gibt dabei die Daten von Hand in das jeweilige Dokumentationstool ein oder trägt diese auf einem Auftragszettel ein. Dies ist mühsam, zeitaufwändig und fehleranfällig. Im Ergebnis führt dies dazu, dass Informationen im Bearbeitungsprozess oft nicht oder nur ungenügend dokumentiert und weitergegeben werden.

Es ist die technische Aufgabe der vorliegenden Erfindung, ein Bearbeitungsgerät zum Bearbeiten einer dentalen Indikation bereitzustellen, mit dem relevante Zustandsdaten auf einfache Weise weitergegeben und dokumentiert werden können.

Diese technische Aufgabe wird durch Gegenstände nach den unabhängigen Ansprüchen gelöst. Technisch vorteilhafte Ausführungsformen sind Gegenstand der abhängigen Ansprüche, der Beschreibung und der Zeichnungen.

Gemäß einem ersten Aspekt wird die technische Aufgabe durch ein Bearbeitungsgerät zum Bearbeiten einer dentalen Indikation gelöst, mit einer elektronischen Zustandserfassungsvorrichtung zum Erfassen von Zustandsdaten des Bearbeitungsgeräts und/oder der dentalen Indikation; und einer elektronischen Erzeugungsvorrichtung zum Erzeugen eines Codes, der die Zustandsdaten umfasst. Eine dentale Indikation ist ein künstlich hergestellter Gegenstand, der im Mund eines Patienten verwendet wird, wie beispielsweise eine Krone, eine Brücke, eine Voll- oder Teilprothese, ein Aligner, eine Knirschschiene, ein Implantat, ein Abutment oder ein Teleskopprothese. Das Bearbeitungsgerät dient zum Bearbeiten oder Herstellen der dentalen Indikation in einem technischen Verfahren. Durch das Bearbeitungsgerät wird der technische Vorteil erreicht, dass die Zustandsdaten auf einfache Weise über den erzeugten Code ausgetauscht und an andere Geräte weitergegeben werden können.

In einer technisch vorteilhaften Ausführungsform des Bearbeitungsgerätes umfasst das Bearbeitungsgerät eine Anzeigevorrichtung zum Anzeigen des Codes. Die Anzeigevorrichtung ist beispielsweise durch ein elektronisches Matrixdisplay gebildet. Dadurch wird beispielsweise der technische Vorteil erreicht, dass der Code direkt an dem Bearbeitungsgerät abgelesen werden kann.

In einer weiteren technisch vorteilhaften Ausführungsform des Bearbeitungsgerätes ist die Anzeigevorrichtung ausgebildet, den Code in einer optisch erfassbaren oder scanbaren Weise anzuzeigen. Dadurch wird beispielsweise der technische Vorteil erreicht, dass der Code automatisch von einem anderen Gerät mit einer Kamera oder einem Scanner optoelektronisch eingelesen werden kann.

In einer weiteren technisch vorteilhaften Ausführungsform des Bearbeitungsgerätes ist der Code ein Bar-Code, ein QR-Code, ein Text-Code oder ein maschinenlesbarer Code. Dadurch wird beispielsweise der technische Vorteil erreicht, dass sich die Zustandsdaten effizient und automatisch erfassbar in den Code integrieren lassen.

In einer weiteren technisch vorteilhaften Ausführungsform des Bearbeitungsgerätes umfasst das Bearbeitungsgerät eine Datenschnittstelle zum elektronischen Versenden des Codes. Die Schnittstelle ist beispielsweise eine drahtlose oder drahtgebundene Datenschnittstelle für ein elektronisches Netzwerk, wie beispielsweise eine Netzwerkarte oder eine WLAN-Schnittstelle. Dadurch wird beispielsweise der technische Vorteil erreicht, dass sich der Code auf elektronischem Weg direkt an andere Geräte übertragen lässt.

In einer weiteren technisch vorteilhaften Ausführungsform des Bearbeitungsgerätes umfasst das Bearbeitungsgerät eine Erfassungsschnittstelle zum optischen Erfassen von Daten eines Herstellungsmaterials. Die Erfassungsschnittstelle ist beispielsweise durch eine elektronische Kamera zum Aufnehmen eines Bildes und ein entsprechendes Scanprogramm zum Scannen des aufgenommenen Bildes gebildet. Dadurch wird beispielsweise der technische Vorteil erreicht, dass sich Daten von verwendeten Herstellungsmaterialien auf einfache und schnelle Weise erfassen lassen und ebenfalls in den Code integrieren lassen.

In einer weiteren technisch vorteilhaften Ausführungsform des Bearbeitungsgerätes umfasst der Code Daten über eine Identifikationsnummer des Bearbeitungsgerätes, über ein Herstellungsmaterial, über eine Anzahl von Betriebsstunden, über eine durchgeführte Kalibration, über eine Fälligkeit der nächsten Kalibration, über Fehlermeldungen und Warnungen, über eine Statistik, über die verwendeten Programme oder Aufträge, über einen laufenden Prozess, über einen Prozessparameter, über Aufträge, die bei der Bearbeitung verarbeitet werden, über Materialien, die bei der Bearbeitung verwendet werden, über eine installierte Softwareversion und/oder über eine fehlerfreie Durchführung eines Prozesses. Dadurch wird beispielsweise der technische Vorteil erreicht, dass Daten für weitere Bearbeitungsschritte oder Dokumentationsschritte in den Code integriert werden.

In einer weiteren technisch vorteilhaften Ausführungsform des Bearbeitungsgerätes ist das Bearbeitungsgerät ein Fräsgerät, ein 3D-Drucker, ein Brennofen, ein Pressofen, ein Sinterofen, ein Scanner, ein Lichthärtegerät, ein Nachbelichtungsgerät, ein Mischsystem, ein Farbmesssystem, ein Polymerisationsgerät, ein Sterilisationsgerät, ein Handstück von Zahntechniker oder ein Reinigungsgerät. Dadurch wird beispielsweise der technische Vorteil erreicht, dass Daten von dentalen Bearbeitungsprozessen in den Code integriert werden.

Gemäß einem zweiten Aspekt wird die technische Aufgabe durch ein Bearbeitungssystem gelöst, mit einem Bearbeitungsgerät nach einem dem ersten Aspekt; und einer Softwareanwendung zum Empfangen des Codes. Die Softwareanwendung kann auf einem Mobiltelefon, einem Tablet-PC, einer Augmented-Reality-Brille oder einem Computer implementiert sein. Dadurch wird der technische Vorteil erreicht, dass der Code auf einfache Weise weitergeleitet und verarbeitet werden kann.

Gemäß einem dritten Aspekt wird die technische Aufgabe durch ein Verfahren zum Bearbeiten einer dentalen Indikation gelöst, mit den Schritten eines Erfassens von Zustandsdaten des Bearbeitungsgeräts und/oder der dentalen Indikation; und eines Erzeugens eines Codes, der die Zustandsdaten umfasst. Dadurch werden die gleichen technischen Vorteile wie durch das Bearbeitungsgerät nach dem ersten Aspekt erreicht.

In einer technisch vorteilhaften Ausführungsform des Verfahrens wird der Code optisch angezeigt oder über eine Datenschnittstelle versendet. Dadurch wird beispielsweise der technische Vorteil erreicht, dass sich der Code schnell und einfach übermitteln lässt.

In einer weiteren technisch vorteilhaften Ausführungsform des Verfahrens werden Daten über eine Identifikationsnummer des Bearbeitungsgerätes, über ein Herstellungsmaterial, über eine Anzahl von Betriebsstunden, über eine durchgeführte Kalibration, über eine Fälligkeit der nächsten Kalibration, über Fehlermeldungen und Warnungen, über eine Statistik der verwendeten Programme oder Aufträge, über einen laufenden Prozess, über einen Prozessparameter, über Aufträge, die bei der Bearbeitung verarbeitet werden, über Materialien, die bei der Bearbeitung verwendet werden, über eine installierte Softwareversion und/oder über eine fehlerfreie Durchführung eines Prozesses erfasst und/oder in den Code eingebettet. Dadurch wird beispielsweise ebenfalls der technische Vorteil erreicht, dass Daten für weiter Bearbeitungsschritte oder Dokumentationsschritte in den Code integriert werden.

In einer weiteren technisch vorteilhaften Ausführungsform des Verfahrens wird der Code an einen Datenknoten übertragen. Der Datenknoten kann beispielsweise ein lokaler oder dezentraler Computer, ein Mobiltelefon oder ein Tablet sein. Der Datenknoten ist beispielsweise über einen Datenkanal mit dem Bearbeitungsgerät verbunden und kann weitere Bearbeitungsschritte auf Basis des Codes durchführen. Dadurch wird beispielsweise der technische Vorteil erreicht, dass Daten für weiter Bearbeitungsschritte oder Dokumentationsschritte in den Code integriert werden.

In einer weiteren technisch vorteilhaften Ausführungsform des Verfahrens wird der Code in einer elektronischen Patientenakte gespeichert. Die Patientenakte kann in digitaler Weise zentral und dezentral gespeichert werden, wie beispielsweise auf einem Internetserver oder einem lokalen PC als Datei oder in einer Datenbank. Die Patientenakte kann dabei manipulationssicher gespeichert werden, indem diese mit einem kryptografischen verfahren digital signiert wird oder als Datensatz in einer Blockchain abgelegt wird. Dadurch wird beispielsweise der technische Vorteil erreicht, dass eine sichere Dokumentation für einen Zahnarzt durchgeführt wird.

In einer weiteren technisch vorteilhaften Ausführungsform des Verfahrens wird eine Plausibilitätsprüfung durchgeführt, ob ein Herstellungsmaterial oder Maschinenparameter für eine vorgegebene dentale Indikation geeignet sind. Bei dieser Plausibilitätsprüfung kann überprüft werden, ob ein Herstellungsmaterial für das Bearbeitungsgerät oder ein bestimmtes Bearbeitungsverfahren geeignet ist. Das Ergebnis dieser Plausibilitätsprüfung kann ebenfalls als Zustandsdaten in den Code integriert werden. Dadurch wird beispielsweise der technische Vorteil erreicht, dass Fehler beim Bearbeitungsprozess verhindert oder erkannt werden können.

Ausführungsbeispiele der Erfindung sind in den Zeichnungen dargestellt und werden im Folgenden näher beschrieben.

Es zeigen:
- Fig. 1: eine schematische Darstellung eines Bearbeitungsgerätes;
- Fig. 2: eine Darstellung eines Herstellungssystem mit einem Bearbeitungsgerät und einer Softwareanwendung zum Empfangen eines Codes;
- Fig. 3: ein Blockdiagramm für ein beispielhaftes Herstellungsverfahren; und
- Fig. 4: ein Blockdiagramm eines Verfahrens zum Bearbeiten einer dentalen Indikation.

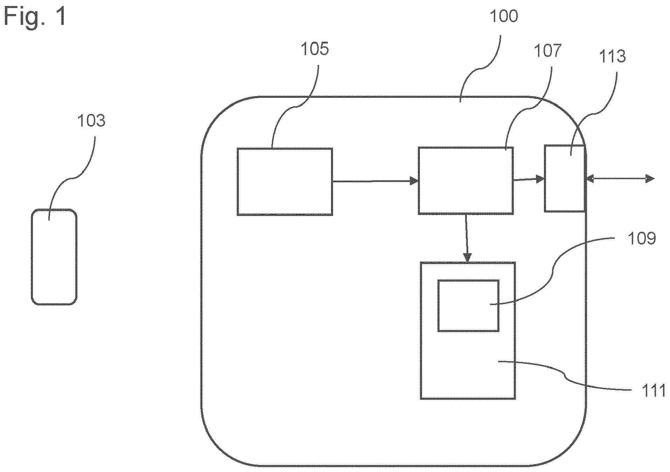

Fig. 1 zeigt eine schematische Darstellung eines Bearbeitungsgerätes 100. Das Bearbeitungsgerät 100 ist ein Gerät, das im Rahmen eines Bearbeitungsverfahrens verwendet wird, mit dem eine dentale Indikation 103 hergestellt oder bearbeitet wird. Hierbei kann es sich beispielsweise um ein Fräsgerät, einen 3D-Drucker, einen Brennofen, einen Pressofen, einen Sinterofen, einen Scanner, ein Lichthärtegerät, ein Nachbelichtungsgerät, ein Mischsystem, ein Farbmesssystem, ein Polymerisationsgerät, ein Sterilisationsgerät, ein Handstück von Zahntechniker oder ein Reinigungsgerät für die dentale Indikation 103 handeln.

Das Bearbeitungsgerät 100 umfasst eine elektronische Zustandserfassungsvorrichtung 105 zum Erfassen von Zustandsdaten des Bearbeitungsgeräts 100 und/oder der dentalen Indikation 103 mit einer geeigneten elektrischen Schaltung und/oder einem Sensor. Zudem kann die Zustandserfassungsvorrichtung 105 einen oder mehrere Sensoren zum Erfassen der Zustandsdaten aufweisen.

Die Zustandsdaten können beispielsweise statische Daten des Bearbeitungsgerätes 100 oder dynamische Daten eines Bearbeitungsverfahrens sein. Durch die elektronische Zustandserfassungsvorrichtung 105 kann beispielsweise eine interne Identifikationsnummer oder ein digitaler Betriebsstundenzähler des Bearbeitungsgeräts 100 ausgelesen werden. Durch die elektronische Zustandserfassungsvorrichtung 105 kann der Zeitpunkt einer durchgeführten Kalibration des Bearbeitungsgeräts 100 bestimmt werden. Durch die elektronische Zustandserfassungsvorrichtung 105 können digitale Informationen über Fehlermeldungen und Warnungen erfasst werden.

Durch die elektronische Zustandserfassungsvorrichtung 105 können eine Statistik über die verwendeten Programme oder Aufträge, Daten über einen laufenden Bearbeitungsprozess und/oder Prozessparameter erfasst werden. Zudem können Daten über Aufträge, die gegenwärtig verarbeitet werden oder über Herstellungsmaterialien erfasst werden, die gegenwärtig verwendet werden. Daneben kann die Zustandserfassungsvorrichtung 105 Daten über eine installierte Softwareversion und/oder über eine fehlerfreie Durchführung eines Prozesses als Zustandsdaten erfassen. Weiter können Daten zum aktuell ausgeführten Prozess als Zustandsdaten des Bearbeitungsgerätes 100 erfasst werden, wie beispielsweise Prozessparameter, bearbeitete Objekte/Aufträge, Beladungszustände, Materialinformationen. Diese Informationen können im Allgemeinen jedoch auch manuell hinzugefügt werden.

Die so erfassten Zustandsdaten werden von der Zustandserfassungsvorrichtung 105 an eine elektronische Erzeugungsvorrichtung 107 übermittelt, die aus den Daten dynamisch einen Code 109 erzeugt. Der Code 109 enthält die jeweils vorgesehenen Zustandsdaten. Der Code 109 ist beispielsweise ein Bar-Code, ein QR-Code, ein Text-Code oder ein maschinenlesbarer Code, der von einem entsprechenden Algorithmus auf Basis der übermittelten Zustandsdaten erzeugt wird. Zu diesem Zweck kann die Erzeugungsvorrichtung 107 durch einen Mikroprozessor oder mittels einer digitalen Schaltung implementiert sein. Der Code 109 kann beispielsweise per Knopfdruck an dem Bearbeitungsgerät erzeugt oder angezeigt werden.

Der Code 109 wird anschließend an eine Anzeigevorrichtung 111 übermittelt und auf dieser angezeigt. Die Anzeigevorrichtung kann beispielsweise ein elektronisches Display sein, auf dem der Code 109 als QR-Code, Bar-Code oder Text-Code angezeigt wird. Der Code 109 kann allerdings auch auf Papier von einem Drucker ausgegeben werden. Von dieser Anzeigevorrichtung 111 kann der Code 109 wiederum von einem anderen elektronischen Gerät eingescannt werden, wie beispielsweise einem Mobiltelefon, einem Tablet-PC oder einer Augmented-Reality-Brille. Außerdem ist es möglich, dass der Code 109 in maschinenlesbarer Form über eine digitale Datenschnittstelle 113 an ein anderes Gerät weitergegeben wird.

Grundsätzlich eignen sich alle Bearbeitungsgeräte 100, die über eine geeignete Anzeigevorrichtung 111 zur optischen Darstellung des Codes 109 verfügen. Sollte die Auflösung der Anzeigevorrichtung 111 nicht groß genug sein, um den Code 109 mit den entsprechenden Informationen anzuzeigen, können die Zustandsdaten auch auf mehrere kleinere Codes 109 aufgeteilt werden, die gleichzeitig oder nacheinander auf der Anzeigevorrichtung 111 erscheinen.

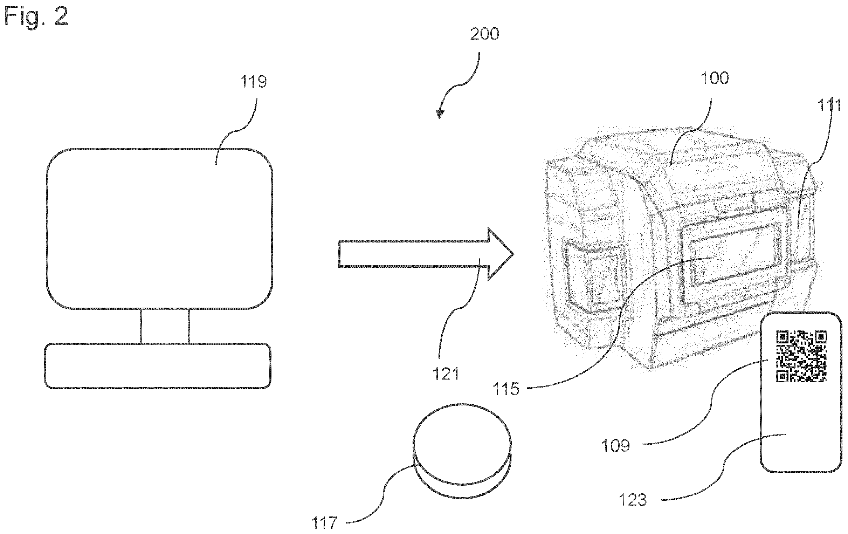

Fig. 2 zeigt eine Darstellung eines Bearbeitungssystems 200 mit dem Bearbeitungsgerät 100 und einer Softwareanwendung zum Empfangen des Codes 109. Der Computer 119 umfasst beispielsweise eine Software, mit der die dentale Indikation 103 von einem Benutzer gestaltet werden kann. Sobald der Datensatz an dem Computer 119 fertiggestellt ist, wird dieser über einen Datenkanal 121 an das Bearbeitungsgerät 100 digital übermittelt.

Das Bearbeitungsgerät 100 bearbeitet die dentale Indikation 103 auf Basis des übermittelten Datensatzes. Hierbei können die oben genannten Zustandsdaten gewonnen werden. Der Code 109, in den die Zustandsdaten eingebettet sind, wird von der Erzeugungsvorrichtung 107 erzeugt und anschließend auf der Anzeigevorrichtung 111 optisch angezeigt.

Dieser Code 109 kann dann von einem geeigneten Gerät 123, wie beispielsweise einem Mobiltelefon, einem Tablet-PC oder einer AR-Brille, wiederum optisch eingelesen werden. Dieses Gerät 123 kann die so übermittelten Zustandsdaten dann aus dem Code 109 extrahieren und weiterverarbeiten. Beispielsweise kann ein Protokoll mit Herstellungsdaten einer im Gerät 123 bearbeiteten dentalen Indikation 103 hinzugefügt werden.

Hierbei kann zudem eine Plausibilitätsprüfung durchgeführt werden, bei der zunächst das Herstellungsmaterial 117 erfasst wird. Dies kann durch einen optischen Scanvorgang über eine Erfassungsschnittstelle 115 erfolgen, mit der die Daten des Herstellungsmaterials 117 erfasst werden. Die Erfassungsschnittstelle 115 ist beispielsweise durch eine elektronische Kamera und eine Bilderkennungssoftware gebildet.

Das Herstellungsmaterial 117 kann beispielsweise auf folgende Weise erkannt werden:
- Schrifterkennung auf dem Etikett (Produktname, LOT-Nummer);
- Farbgebung des Etiketts oder der Verpackung;
- Vergleich des Etiketts mit einem Etikett, das aus einer Datenbank abgerufen wird; und/oder
- Form der Verpackung.

Anschließend wird im Rahmen der Plausibilitätsprüfung geprüft, ob das erfasste Herstellungsmaterial 117 auch für die dentale Indikation 103 geeignet ist und von dem Bearbeitungsgerät 100 in der gewünschten Art und Weise bearbeitet werden kann.

Hierzu können die jeweiligen Maschinenparameter des Bearbeitungsgeräts 100 als Zustandsdaten berücksichtigt werden. Verläuft die Plausibilitätsprüfung mit positivem Ergebnis, erfolgt eine Freigabe der Bearbeitung durch das Bearbeitungsgerät 100. Die so erhaltenen Zustandsdaten der Plausibilitätsprüfung können ebenfalls in den Code 109 eingebettet werden. Zudem können Informationen über das gegenwärtig verwendete Herstellungsmaterial 117 und weitere dem Bearbeitungsgerät 100 bekannte Informationen als Zustandsdaten erfasst und ausgelesen werden, wie beispielsweise ein Gerätestatus mit Kalibration-, Service- und Softwareupdate-Informationen und weitere Messwerte. Auch diese Zustandsdaten können in dem Code 109 hinterlegt und in diesen eingebettet werden.

Weitere Zustandsdaten können unter anderem Informationen umfassen, über:
- eine Nummer zur Identifikation des jeweiligen Bearbeitungsgerätes (IOS-Daten, Gerätenummer, SoftwareVersion, Hardware-Version);
- einen Gerätestatus;
- ein verwendetes Herstellungsmaterial (LOT-Nummer, Materialbezeichnung, Materialinformation, Schichtprozess);
- eine Anzahl Betriebsstunden des Bearbeitungsgerätes;
- einen Zeitpunkt (Datum/Uhrzeit) der letzten Kalibration des Bearbeitungsgerätes;
- einen Zeitpunkt (Datum/Uhrzeit) der Fälligkeit der nächsten Kalibration;
- ein Protokoll (Log) der Prozesse, Fehlermeldungen und Warnungen;
- eine Statistik über die bei der Bearbeitung verwendeten Programme oder Aufträge;
- eine Information über einen gegenwärtig laufenden Prozess oder Prozessparameter;
- einen Auftrag, der gegenwärtig verarbeitet wird;
- Patientendaten;
- Bearbeiterdaten;
- Benutzerdaten;
- Herstellerdaten (Zahnpraxis; Zahnlabor);
- eine Auftragsbeschreibung (Art der dentalen Indikation, Farbe, Material; Größe; Menge);
- ein Datenformat (STL - Standard Tesselation Language)
- eine CAM-Materialinformation (Bezeichnung, Werkzeuge, LOT-Nr.) ;
- eine CAM-Geräteinformationen (Identifikationsnr., Gerätestatus, Kalibrationsstatus);
- CAM-Prozessinformationen (Frässtrategie, Bauparameter, CAM-Softwareversion) ;
- eine digitale Einprobe (Farbe);
- einen Öffnungszustand einer Tür eines Fräsraums (offen/geschlossen) ;
- einen Öffnungszustand eines Ventils (offen/geschlossen);
- einen Werkzeugzustand (vorhanden/gebrochen); und/oder
- einen Materialzustand (vorhanden/eingelegt).

Sensordaten können Informationen umfassen, über:
- physikalische Größen (Drehzahl, Temperatur, Druck, Frequenz);
- chemische Größen;
- Ableitungen der chemischen oder physikalischen Größen über die Zeit (relativ oder absolut);
- Standortdaten oder Positionsdaten;
- eine Identifikation (RFID, Barcode);
- einen Status und Signale (An, Aus); und/oder
- einen Zeitstempel.

Datenformate der Sensoren umfassen:
- Zahlen;
- Text;
- Bild (2D, 3D);
- Video (Geste);
- Audio (Sprache); und/oder
- Status (True, False).

Aufgaben, die mit Hilfe der Sensoren oder Sensordaten ausgeführt werden, umfassen:
- Steuerung und Planung (Control and Planning);
- Verfolgung (Track and Trace);
- Visuelle Inaugenscheinnahme (Visual Inspection);
- Dokumentation;
- Führung und Hilfe (Guide and Help);
- Unterstützung (Support); und/oder
- Werkzeuge und Eigenschaften (Tools and Features, wie beispielsweise eine Lupe).

Bei einer fehlerfreien Durchführung eines Prozesses, wird der Code 109 erst am Ende des durchgeführten Prozesses ausgegeben. Wenn das Bearbeitungsgerät 100 nicht mehr konform ist, beispielsweise eine Kalibration fehlschlägt, wird eine Warnung ausgegeben und Code 109 nicht mehr angezeigt.

Auf diese Weise wird die einfache und vollständige Dokumentation aller relevanten Geräteparameter ermöglicht, indem der dynamische und/oder statischen Codes erfasst wird, der alle benötigten Informationen enthält.

Zudem kann eine automatische elektronische Nachbestellung der verbrauchten Herstellungsmaterialien 117 erfolgen oder ein Risikomanagement auf Basis der Zustandsdaten durchgeführt werden. Es können Statistiken über Aufträge und ein Materialverbrauch erhalten werden. Durch die Zustandsdaten wird eine Rückverfolgbarkeit und Nachvollziehbarkeit des Herstellungsprozesses erreicht, beispielsweise anhand von Chargennummern und einem Gerätestatus.

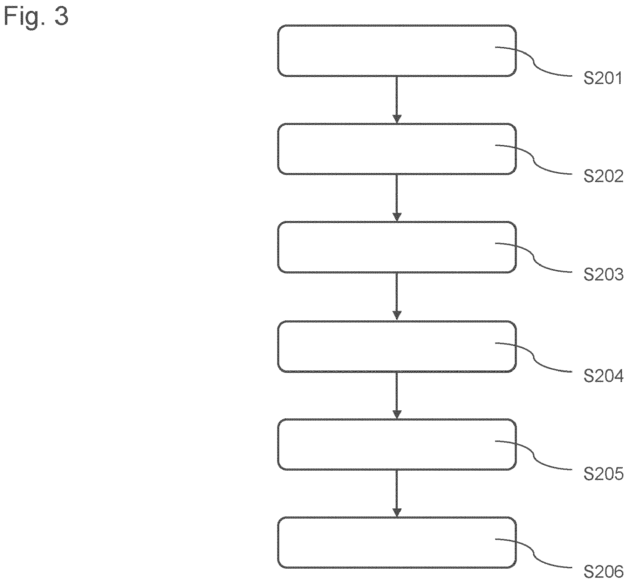

Fig. 3 zeigt ein Blockdiagramm für ein beispielhaftes Herstellungsverfahren für eine dentale Indikation.

In Schritt 201 werden zunächst die Patientendaten beim Zahnarzt erfasst, wie beispielsweise ein Kieferabdruck, ein Intraoralscan oder eine Zahnfarbe, und es wird ein Auftrag für den Zahntechniker erstellt. Der Zahntechniker enthält neben den Patientendaten möglicherweise weitere Informationen über das zu verwendende Material, wie beispielsweise Metallkeramik oder Zirkonoxid.

In Schritt S202 werden diese Informationen vom Zahnarzt zum Zahntechniker übermittelt und eine Dokumentation für den Patientenfall angelegt oder eine bereits vorhandene Dokumentation des Zahnarztes weiterverwendet. Dies kann zum Beispiel funktionieren, wenn der Zahnarzt und der Zahnarzt eine gemeinsame Software verwenden, die die gemeinsame Dokumentation des Patientenfalles erlaubt. Diese Software kann auf einem mobilen Gerät wie einem Mobiltelefon, einem Tablet-PC oder einer Augmented-Reality-Brille (AR-Brille) ausgeführt werden.

In Schritt S203 kann der Zahntechniker zum Beispiel aufgrund der Daten am Computer eine dentale Kappe als dentale Indikation 103 aus Zirkondioxid gestalten, die auf einer Fräse als Bearbeitungsgerät 100 hergestellt wird. Nach Abschluss der Herstellung kann zum Beispiel ein Barcode angezeigt werden, der Informationen in den Zustandsdaten wie das gewählte Material, die minimale Wandstärke und weitere Parameter der dentalen Kappe oder des Bearbeitungsgerätes 100 als Zustandsdaten umfasst. Dieser Barcode kann vom Zahntechniker mittels eines Mobiltelefons eingelesen werden und die Informationen mittels eines Dokumentationstools dem Auftrag hinzugefügt werden.

Auf der Fräse entstehen beim Herstellungsprozess weitere Zustandsdaten. Beispielsweise werden die eingesetzten Werkzeuge und das Material erkannt. Nach Abschluss des Fräsvorgangs können über einen QR-Code diese Informationen und weitere Informationen zum Prozess, wie beispielsweise aufgetretene Fehlermeldungen oder Warnungen oder eine eindeutige Identifikationsnummer der Fräse eingelesen und dem Auftrag über den QR-Code als Zustandsdaten hinzugefügt werden.

Im Schritt S204 wird die dentale Kappe von einem Block abgetrennt, verschliffen und anschließend gesintert. In einem Sinterofen werden wiederum relevante Informationen wie eine eindeutige Identifikation des Sinterofens, das gewählte Sinterprogramm, tatsächlich gemessene Temperaturen, Fehlermeldungen erfasst und im Code 109 hinterlegt.

Im Schritt S205 wird die Restauration beispielsweise mit der Schichttechnik weiter aufgebaut. Hier können ebenfalls verwendete Materialien, wie beispielsweise ein Keramikpulver und die Informationen erfasst werden, wie diese gebrannt worden sind. Hierzu kann ein QR-Code auf dem Sinterofen mit den Informationen des Sinterofens angezeigt werden. Die Erkennung der Materialien kann über einen QR-Code erfolgen, der auf der Verpackung oder dem Material selbst angebracht ist, wie beispielsweise auf dem Pressrohling. Sollte kein Code vorhanden sein, kann das Material an folgenden Merkmalen oder einer entsprechenden Kombination erkannt werden:
- Schrifterkennung auf dem Etikett (Produktname, LOT-Nummer);
- Farbgebung des Etiketts oder Verpackung;
- Vergleich des Etiketts mit einer Datenbank; und/oder
- Form der Verpackung.

In Schritt S206 werden die über den Code 109 bereitgestellten gesammelten Informationen in dem Dokumentationstool zusammengefasst. Dadurch ergibt sich eine vollständige sichere, und papierlose Dokumentation der jeweiligen dentalen Indikation, die dem Zahnarzt für die Eingliederung und (ggf. in reduziertem Umfang) zur Weitergabe an den Kunden zur Verfügung gestellt werden kann. Die Dokumentation kann in eine elektronische Patientenakte integriert werden.

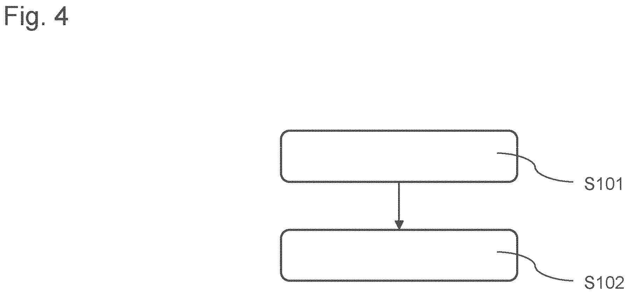

Fig. 4 zeigt ein Blockdiagramm eines Verfahrens zum Bearbeiten der dentalen Indikation. Im Schritt S101 werden zunächst die Zustandsdaten des Bearbeitungsgeräts und/oder der dentalen Indikation erfasst. Im Schritt S102 wird der Code 109 elektronisch erzeugt, der die Zustandsdaten umfasst.

Existierende Bearbeitungsgeräte 100 können, ohne dass eine weitere Schnittstelle nachgerüstet werden muss, über eine Änderung der Software zwecks Datenerfassung und Dokumentation auch nachträglich in das Verfahren eingebunden werden. Grundsätzlich kann bei vorhandener Anzeigevorrichtung 111 für den Code 109 auf aufwändige Schnittstellenbauteile verzichtet werden. Das kann auch von Vorteil sein, wenn batteriebetriebenes Bearbeitungsgerät verbrauchsoptimiert arbeiten soll. Um ein älteres Bearbeitungsgerät 100 in das Verfahren einzubinden, wird lediglich ein Softwareupdate benötigt, in dem definiert ist, welche Informationen im Code 109 hinterlegt sind, wie groß der Code 109 ist, welches Grunddesign dieser aufweist und wann dieser angezeigt wird.

Das Verfahren kann für eine Dokumentationspflicht in allen medizinischen Bereichen und damit auch im Dentalbereich für den Zahnarzt und beim Zahntechniker eingesetzt werden, da hierbei die Zustandsdaten des Bearbeitungsgerätes 100 eine wichtige Rolle spielen, mit dem die dentale Indikation 103 bearbeitet wird. Zudem ergibt sich eine Zeitersparnis für eine einfache und vollständige Dokumentation der Zustandsdaten.

Das Verfahren kann auch bei Bearbeitungsgeräten 100 verwendet werden, die keine Schnittstelle für die Einbindung zu übergeordneten Systemen haben. Allerdings können die Bearbeitungsgeräte 100 über eine Schnittstelle, wie beispielsweise kabellos (WLan oder Bluetooth) oder über Kabel (Lan) eingebunden werden, über die eine Übertragung des Codes in ein übergeordnetes System erfolgt.

Alternative Möglichkeiten sind, dass das Bearbeitungsgerät 100 eine Protokolldatei führt, die Auskunft über die Abarbeitung von Aufträgen gibt oder über einen angeschlossenen Drucker bei Abarbeitung eines Auftrags ein Dokument für eine papierbasierte Dokumentation ausgibt.

Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Alle Verfahrensschritte können durch Vorrichtungen implementiert werden, die zum Ausführen des jeweiligen Verfahrensschrittes geeignet sind. Alle Funktionen, die von gegenständlichen Merkmalen ausgeführt werden, können ein Verfahrensschritt eines Verfahrens sein.

Der Schutzbereich der vorliegenden Erfindung ist durch die Ansprüche gegeben und wird durch die in der Beschreibung erläuterten oder den Figuren gezeigten Merkmale nicht beschränkt.

### BEZUGSZEICHENLISTE

- 100: Bearbeitungsgerät
- 103: dentale Indikation
- 105: Zustandserfassungsvorrichtung
- 107: Erzeugungsvorrichtung
- 109: Code
- 111: Anzeigevorrichtung
- 113: Datenschnittstelle
- 115: Erfassungsschnittstelle
- 117: Herstellungsmaterial
- 119: Computer
- 121: Datenkanal
- 123: Gerät

## Patentansprüche

1. Bearbeitungsgerät (100) zum Bearbeiten einer dentalen Indikation (103), mit:
- einer elektronischen Zustandserfassungsvorrichtung (105) zum Erfassen von Zustandsdaten des Bearbeitungsgeräts (100) und/oder der dentalen Indikation (103); und
- einer elektronischen Erzeugungsvorrichtung (107) zum Erzeugen eines Codes (109), der die Zustandsdaten umfasst.

2. Bearbeitungsgerät (100) nach Anspruch 1, wobei das Bearbeitungsgerät (100) eine Anzeigevorrichtung (111) zum Anzeigen des Codes (109) umfasst.

3. Bearbeitungsgerät (100) nach einem der vorangehenden Ansprüche, wobei die Anzeigevorrichtung (111) ausgebildet ist, den Code (109) in einer optisch erfassbaren oder scanbaren Weise anzuzeigen.

4. Bearbeitungsgerät (100) nach einem der vorangehenden Ansprüche, wobei der Code ein Bar-Code, ein QR-Code, ein Text-Code oder ein maschinenlesbarer Code ist.

5. Bearbeitungsgerät (100) nach einem der vorangehenden Ansprüche, wobei das Bearbeitungsgerät (100) eine Datenschnittstelle (113) zum elektronischen Versenden des Codes (109) umfasst.

6. Bearbeitungsgerät (100) nach einem der vorangehenden Ansprüche, wobei das Bearbeitungsgerät (100) eine Erfassungsschnittstelle (115) zum optischen Erfassen von Daten eines Herstellungsmaterials (117) umfasst.

7. Bearbeitungsgerät (100) nach einem der vorangehenden Ansprüche, wobei der Code Daten über eine Identifikationsnummer des Bearbeitungsgerätes, über ein Herstellungsmaterial, über eine Anzahl von Betriebsstunden, über eine durchgeführte Kalibration, über eine Fälligkeit der nächsten Kalibration, über Fehlermeldungen und Warnungen, über eine Statistik der verwendeten Programme oder Aufträge, über einen laufenden Prozess, über einen Prozessparameter, über Aufträge, die bei der Bearbeitung verarbeitet werden, über Materialien, die bei der Bearbeitung verwendet werden, über eine installierte Softwareversion und/oder über eine fehlerfreie Durchführung eines Prozesses umfasst.

8. Bearbeitungsgerät (100) nach einem der vorangehenden Ansprüche, wobei das Bearbeitungsgerät ein Fräsgerät, ein 3D-Drucker, ein Brennofen, ein Pressofen, ein Sinterofen, ein Scanner, ein Lichthärtegerät, ein Nachbelichtungsgerät, ein Mischsystem, ein Farbmesssystem, ein Polymerisationsgerät, ein Sterilisationsgerät, ein Handstück von Zahntechniker oder ein Reinigungsgerät ist.

9. Bearbeitungssystem (200), mit:
- einem Bearbeitungsgerät (100) nach einem der Ansprüche 1 bis 9; und
- einer Softwareanwendung zum Empfangen des Codes (109) .

10. Verfahren zum Bearbeiten einer dentalen Indikation (103), mit den Schritten:
- Erfassen (S101) von Zustandsdaten des Bearbeitungsgeräts (100) und/oder der dentalen Indikation (103); und
- Erzeugen (S102) eines Codes (109), der die Zustandsdaten umfasst.

11. Verfahren nach Anspruch 10, wobei der Code (109) optisch angezeigt wird oder über eine Datenschnittstelle (113) versendet wird.

12. Verfahren nach einem der Ansprüche 10 oder 11, wobei Daten über eine Identifikationsnummer des Bearbeitungsgerätes, über ein Herstellungsmaterial, über eine Anzahl von Betriebsstunden, über eine durchgeführte Kalibration, über eine Fälligkeit der nächsten Kalibration, über Fehlermeldungen und Warnungen, über eine Statistik über die verwendeten Programme oder Aufträge, über einen laufenden Prozess, über einen Prozessparameter, über Aufträge, die bei der Bearbeitung verarbeitet werden, über Materialien, die die bei der Bearbeitung verwendet werden, über eine installierte Softwareversion und/oder über eine fehlerfreie Durchführung eines Prozesses erfasst und/oder in den Code (109) eingebettet werden.

13. Verfahren nach einem der Ansprüche 10 bis 12, wobei der Code (109) an einen Datenknoten übertragen wird.

14. Verfahren nach einem der Ansprüche 10 bis 13, wobei der Code (109) in einer elektronischen Patientenakte gespeichert wird.

15. Verfahren nach einem der Ansprüche 10 bis 14, wobei eine Plausibilitätsprüfung durchgeführt wird, ob ein Herstellungsmaterial (117) oder Maschinenparameter für eine vorgegebene dentale Indikation geeignet sind.
